Europäisches Patentamt

European Patent Office

Office européen des brevets

(19)

(11) Veröffentlichungsnummer: **0 114 617**
**B1**

(12) **EUROPÄISCHE PATENTSCHRIFT**

(45) Veröffentlichungstag der Patentschrift:
22.03.89

(21) Anmeldenummer: 84100254.6

(22) Anmeldetag: 12.01.84

(51) Int. Cl.⁴: **B 05 B 11/00**, B 65 D 47/34,
F 04 B 9/14, G 06 M 1/04,
A 61 J 7/00, G 01 F 13/00

(54) **Betätigbare Dosiereinrichtung.**

(30) Priorität: 22.01.83 DE 3302160

(43) Veröffentlichungstag der Anmeldung:
01.08.84 Patentblatt 84/31

(45) Bekanntmachung des Hinweises auf die Patenterteilung:
22.03.89 Patentblatt 89/12

(84) Benannte Vertragsstaaten:
AT BE CH DE FR GB IT LI NL SE

(56) Entgegenhaltungen:
GB-A- 1 317 315
GB-A- 2 063 075
US-A- 4 009 370
US-A- 4 162 746

(73) Patentinhaber: Ing. Erich Pfeiffer GmbH & Co. KG,
Josef-Bosch-Strasse 4, D-7760 Radolfzell (DE)

(72) Erfinder: Pfeiffer, Peter, Gütebohlweg 12,
D-7766 Gaienhofen (DE)
Erfinder: Märte, Leo, Rathausstrasse 25,
D-7767 Sipplingen (DE)

(74) Vertreter: Patentanwälte Ruff und Beier,
Neckarstrasse 50, D-7000 Stuttgart 1 (DE)

## Beschreibung

Die Erfindung betrifft eine betätigbare Dosiereinrichtung mit einem Betätigungsdrücker zur Ausgabe einer Mengeneinheit eines fließfähigen Stoffes bei jedem Betätigungshub aus einem Behälter und mit einer, insbesondere einen Zählring aufweisenden, Zähleinrichtung für die Betätigungshübe.

Bei einer bekannten Dosiereinrichtung (GB-A-1 317 315) dieser Art weist die Zähleinrichtung eine optische Zählskala auf, anhand von welcher die Anzahl der jeweils durchgeführten Betätigungshübe festzustellen und nach Erreichen der gewollten Anzahl zu beenden ist. Diese bekannte Dosiereinrichtung vermag daher nichts weiter zu bewirken, als die Anzahl der Betätigungshübe zu zählen, ohne daß hiervon eine weitere Funktion abgeleitet werden könnte. Die nur mit der sichtbaren Zählskalierung sinnvolle Dosiereinrichtung ist so ausgebildet, daß sie nur für kurze Betätigungshübe geeignet ist, wie sie am Betätigungsventil eines treibmittelgefüllten Behälters üblich sind, weshalb die bekannte Dosiereinrichtung für die aus Umweltschutzgründen zu bevorzugenden Austragvorrichtungen mit Schubkolbenpumpen oder dgl. nicht geeignet ist.

Der Erfindung liegt die Aufgabe zugrunde, eine betätigbare Dosiereinrichtung der genannten Art zu schaffen, mit der bei einer beabsichtigten Beendigung der Anwendungsdauer nach einer bestimmten Anzahl von Betätigungshüben die Dosiereinrichtung blockiert und ggf. eine veränderliche Dosierung einfach und sinnfällig vorgenommen werden kann.

Diese Aufgabe wird bei einer betätigbaren Dosiereinrichtung der eingangs beschriebenen Art gemäß der Erfindung dadurch gelöst, daß eine in Abhängigkeit von der Zähleinrichtung betätigbare Hubsperre für den Betätigungshub vorgesehen ist. Dadurch braucht die Anzahl der dosierten Stoffausträge bei einer z.B. zeitlich limitierten Austragdauer nicht zwingend aufmerksam anhand einer Zählskalierung verfolgt zu werden, sondern nach dem letzten der zahlenmäßig bestimmten Betätigungshübe dieser Austragsfolge wird die Dosiereinrichtung entweder endgültig oder wieder auslösbar, jedoch zumindest so, gesperrt, daß der Benutzer ein Signal für die Beendigung eines Austragzyklus' erhält.

Derartige Dosiereinrichtungen können für die verschiedensten Anwendungszwecke und Ausgabemedien verwendet werden, unter anderem auch für medizinische Zwecke. Es kann sich dabei um Dosier- oder Zerstäuberpumpen handeln, die bei jedem Pumpenhub eine Mengeneinheit des Materials fördern und ausgeben sowie ggf. zerstäuben. Es können jedoch als Dosiereinrichtung auch Dosierventile benutzt werden, die an Behältern mit einem Innendruck angebracht sind und bei Betätigung zur Ausgabe einer bestimmten Menge öffnen und danach wieder schließen. Die bei einem Betätigungshub ausgegebenen Mengeneinheiten können bei geeigneter Ausbildung der Dosiereinrichtung in relativ engen Grenzen konstant gehalten werden, so daß auch eine Dosierung von solchen Pharmazeutika möglich ist, deren Wirkung von der Dosierung abhängig ist. Durch die erfindungsgemäße Ausbildung läßt sich jedoch vermeiden, daß versehentlich durch eine zu geringe oder zu große Zahl von Betätigungshüben unter- bzw. überdosiert wird oder daß die Anwendung über eine zu lange Zeit oder in der falschen Aufeinanderfolge erfolgt.

Die Sperre für den Betätigungshub kann durch zusammenwirkende Vorsprünge bzw. Flächen an Betätigungsdrücker und Zählring ausgebildet sein. Wenn der Zählring also seine vorgegebene Endlage erreicht hat, wird die Sperre wirksam und der Betätigungshub verhindert.

Die erfindungsgemäße Zähleinrichtung wird von der Betätigung des Betätigungsdrückers ausgelöst und kann in verschiedenartiger Weise ausgebildet sein. Sie kann beispielsweise eine optische Anzeige enthalten, die die Zahl der Betätigungshübe anzeigt.

Nach einem weiteren Merkmal der Erfindung weist die Hubsperre einen über ein Fortschaltwerk der Zähleinrichtung von mindestens einer Ausgangsstellung über Freigabestellungen in eine dem Ende des mehrhubigen Benutzungszyklus' zugehörige Sperrlage bewegten Anschlag auf, dem ein Gegenanschlag des Betätigungsdrückers zugeordnet ist. Dadurch ergibt sich eine besonders einfache Ausbildung, die noch dadurch verbessert werden kann, daß der Anschlag an einem Innenumfang liegt bzw. daß der Gegenanschlag in einer Sperr-Drehlage von dem Anschlag gegen Axialbewegungen gesperrt ist.

Vorteilhaft ist der Anschlag am Zählring, insbesondere in Form eines Vorsprunges, vorgesehen, wobei er bevorzugt am äußeren Ende des Zählringes liegen kann. Durch die erfindungsmäße Ausbildung läßt sich die Dosiereinrichtung auch mit einer üblichen kleinen Hand-Zerstäuberpumpe konstruktiv vorteilhaft vereinigen.

In weiterer Ausgestaltung der Erfindung ist der Anschlag in der Sperr-Drehlage gegen Weiterdrehung durch eine Drehsperre gesperrt, wobei vorzugsweise eine Anschlagfläche, insbesondere die Anschlagfläche des Anschlages, nach Art einer abgesetzten Schulter in einen über sie vorstehenden Drehsperr-Anschlag bzw. eine Drehsperr-Rast übergeht. Durch die erfindungsgemäße Ausbildung kann beispielsweise bei einer beabsichtigten Beendigung der Anwendungsdauer nach einer bestimmten Anzahl von Betätigungshüben, d.h. z.B. von Anwendungstagen, die Dosiereinrichtung blockiert werden, um eine zeitliche Überdosierung zu vermeiden. Auf diese Weise ist es möglich, auch sehr dosierungsabhängige Pharmazeutika in Form von Sprays zu verwenden, bei denen eine genaue Folge von Anwendungstagen einer entsprechenden Zahl von Anwendungspausen gegenüberstehen muß.

Eine besonders vorteilhafte Weiterbildung des Erfindungsgegenstandes besteht darin, daß die Hubsperre lösbar ausgebildet ist und insbesondere nach einer Skalierung in unterschiedliche Dosierungen überführbar ist. Das Vorsehen einer lösbaren Sperre ermöglicht die Anwendung ohne die Notwendigkeit des Mitzählens beim Anwender, indem beispielsweise die Sperre bei einer täglichen Maximaldosierung von einer gewissen Anzahl von Hüben wirksam wird, dann aber wieder gelöst werden kann. Bei einer lösbaren Sperre könnte am Zählring eine Handhabe vorgesehen sein, mit der dieser in eine neue Ausgangslage zu drehen ist. Außerdem ist es durch die erfindungsgemäße Ausbildung möglich, veränderliche Dosierungen einfach und sinnfällig vorzunehmen, indem die jeweilige dieser unterschiedlichen Dosierungen durch eine entsprechende Skalierung und/oder Sperren, die ggf. auch lösbar sein können, vorgegeben wird. So kann die Dosiereinrichtung bereits für eine im Laufe der Behandlungsdauer abnehmende Dosierung vorgesehen sein.

Nach einem weiteren Merkmal der Erfindung ist der Anschlag gegenüber dem Gegenanschlag, insbesondere mit einer Handhabe, aus der Sperrlage in eine Ausgangslage für einen weiteren Benutzungszyklus überführbar, wobei vorzugsweise die Drehsperr-Rast manuell überfahrbar ausgebildet ist. Dadurch kann die Dosiereinrichtung auf einfache Weise auf den Beginn eines wiederholt durchzuführenden Benutzungszyklus eingestellt werden, ohne daß sie auf die Ausgangslage zurückgedreht werden muß.

Insbesondere bei der zuletzt beschriebenen Ausbildung ist es auch möglich, die Zähleinrichtung gegen Rückdrehung, insbesondere durch ein Freilauf-Gesperre, zu sperren, das vorzugsweise durch das Fortschaltwerk gebildet ist. Durch das einseitig wirkende Gesperre läßt sich auf einfache Weise eine unzulässige Rückdrehung in jeder Stellung der Zähleinrichtung bewirken. Statt einer Ausrichtung des Gesperres gleichzeitig als eine gewisse federnde Hemmung zum Verhindern einer unbeabsichtigten Weiterdrehung könnte jedoch auch ein Fortschaltwerk Verwendung finden, bei dem eine formschlüssige Sperrung gegen Weiterdrehung vorliegt.

Bei einer einfachen und sehr funktionssicheren Ausgestaltung des Erfindungsgegenstandes weist das Fortschaltwerk abgeschrägte Schaltflächen und vorzugsweise eine Einrasteinrichtung auf, die den Anschlag bzw. den Zählring jeweils nach Überschreiten einer vorgegebenen Drehstellung jeweils in eine bestimmte Lage weiterdreht. Hierbei braucht also das Fortschaltwerk nicht den vollen Schaltweg durchzuführen, sondern die Weiterdrehung in die jeweilige Folgelage wird durch die Einrasteinrichtung bewirkt und bis zur nächsten Betätigung nach Art einer Rastung festgelegt. Durch die abgeschrägten Schaltflächen ergibt sich ein einfacher Weitertransport des Anschlages bzw. des Zählringes.

Nach einem weiteren Vorschlag gemäß der Erfindung ist ein beispielsweise der Befestigung am Behälter dienender Basisteil vorgesehen, gegenüber welchem der Betätigungsdrücker axial bewegbar und auf dem der ihn umgebende Zählring drehbar gelagert ist, so daß die Dosiereinrichtung dadurch eine besonders einfache und vorteilhafte Ausbildung erhält, daß sie im wesentlichen nur den Basisteil, den Betätigungsdrücker und den Zählring aufweist, wobei zwischen dem Betätigungsdrücker und dem Zählring das bei Axialverschiebung schrittweise arbeitende Fortschaltwerk vorgesehen sein kann. Des weiteren kann der Betätigungsdrücker mit dem Basisteil vorteilhaft drehfest, jedoch axial bewegbar, verbunden sein.

Eine andere besonders vorteilhafte Weiterbildung des Erfindungsgegenstandes besteht darin, daß ein Zusatzring vorgesehen ist, der in den Zählring einkuppelbar ist. Durch diesen Zusatzring kann zusätzlich zu einer Skala eine zweite Skalierung vorgesehen werden, beispielsweise zusätzlich zu einer Datumsfolge eine Wochentagsfolge. Es ist daher möglich, daß beispielsweise durch den vorzugsweise mit einer Skalierung versehenen Zählring bei einer täglich einmaligen Anwendung über eine bestimmte Anzahl von Tagen dieser Zählring die Tagesanzahl zählt, während der Zusatzring den jeweils zugehörigen Wochentag anzeigt. Dadurch kann der Anwender jeweils auch feststellen, ob er an dem betreffenden Tag die Anwendung schon vorgenommen hat oder nicht.

Merkmale von bevorzugten Weiterbildungen der Erfindung gehen aus den Unteransprüchen und der Beschreibung im Zusammenhang mit den Zeichnungen hervor, wobei die einzelnen Merkmale jeweils für sich allein oder zu mehreren in Form von Unterkombinationen bei einer Ausführungsform der Erfindung verwirklicht sein können. Ausführungsbeispiele der Erfindung sind in den Zeichnungen dargestellt und werden im Folgenden näher erläutert. In den Zeichnungen zeigen:

Fig. 1 einen axialen Teilschnitt einer Dosiereinrichtung an einer Zerstäuberpumpe,

Fig. 2 einen Teilschnitt nach der Linie II in Fig. 1,

Fig. 3 einen Teilschnitt nach der Linie III in Fig. 1,

Fig. 4 einen Querschnitt nach der Linie IV, jedoch ohne Pumpeninnenteil,

Fig. 5 einen Teilschnitt nach der Linie V in Fig. 1, ebenfalls ohne Pumpeninnenteil,

Fig. 6 eine Ansicht der Zerstäuberpumpe nach Fig. 1,

Fig. 7 einen axialen Teilschnitt durch eine weitere Ausführungsform,

Fig. 8 einen Teilschnitt nach der Linie VIII in Fig. 7 und

Fig. 9 einen Teilschnitt nach der Linie IX in Fig. 7, jedoch ohne Pumpeninnenteil.

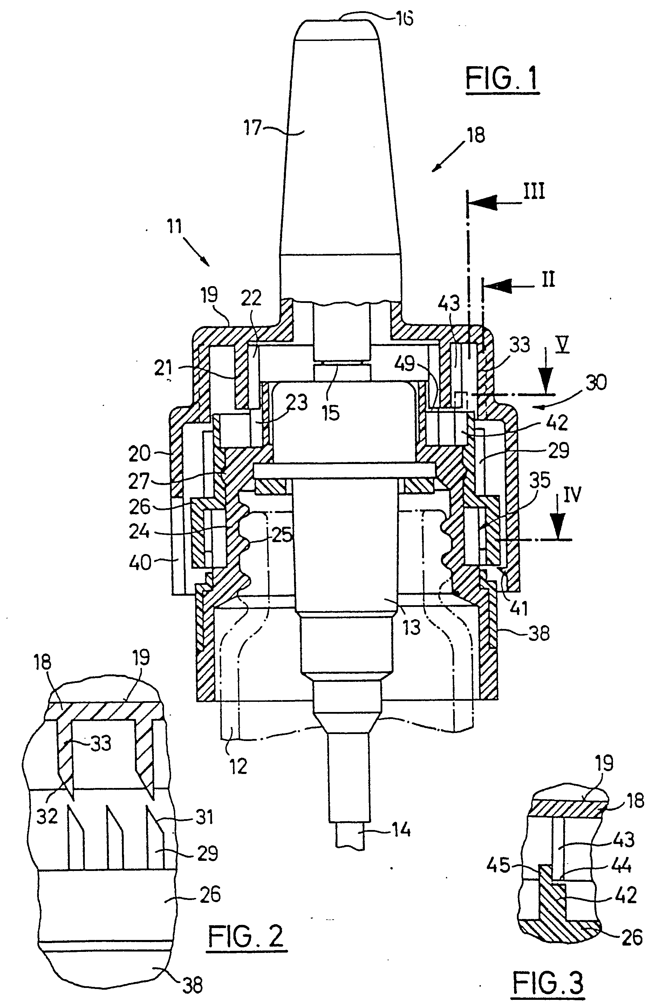

Die Ausführungsform nach den Figuren 1 bis 6 zeigt eine Dosiereinrichtung 11 in Form eines Pumpenzerstäubers, der auf einen strichpunktiert gezeichneten Behälter 12 aufgeschraubt ist. Dieser Pumpenzerstäuber hat eine im einzelnen nicht dargestellte Pumpenkammer 13, in die über einen Saugschlauch 14 Flüssigkeit aus dem Behälterinneren angesaugt wird und unter der Wirkung eines in der Pumpenkammer 13 laufenden Kolbens, der mit einer Kolbenstange 15 verbunden ist, angesaugte Flüssigkeit über eine Zerstäuberdüse 16 ausgibt, die am oberen Ende eines leicht konischen Schaftes 17 angeordnet ist.

Der Schaft gehört zu einem Kunststoff-Bauteil, das einen Betätigungsdrücker 18 zur Betätigung der Pumpe bildet. Er hat eine breite Schulter 19, die den Schaft 17 umgibt und eine Betätigungsfläche für die Finger des Benutzers bildet. Von dort aus ragt ein stufig abgesetzter Mantel 20 abwärts, der die inneren Teile des Pumpenzerstäubers weitgehend umgibt. Von der Schulter 19 ragt ein Ring 21 abwärts, der an seiner Innenseite eine axial, d.h. vertikal verlaufende Verzahnung 22 trägt, die mit einer entsprechenden Verzahnung 23 eines Pumpen-Basisteils 24 in Eingriff ist und eine Axialführung für den Betätigungsdrücker bildet, d.h. axiale Bewegungen erlaubt, jedoch Drehbewegungen verhindert.

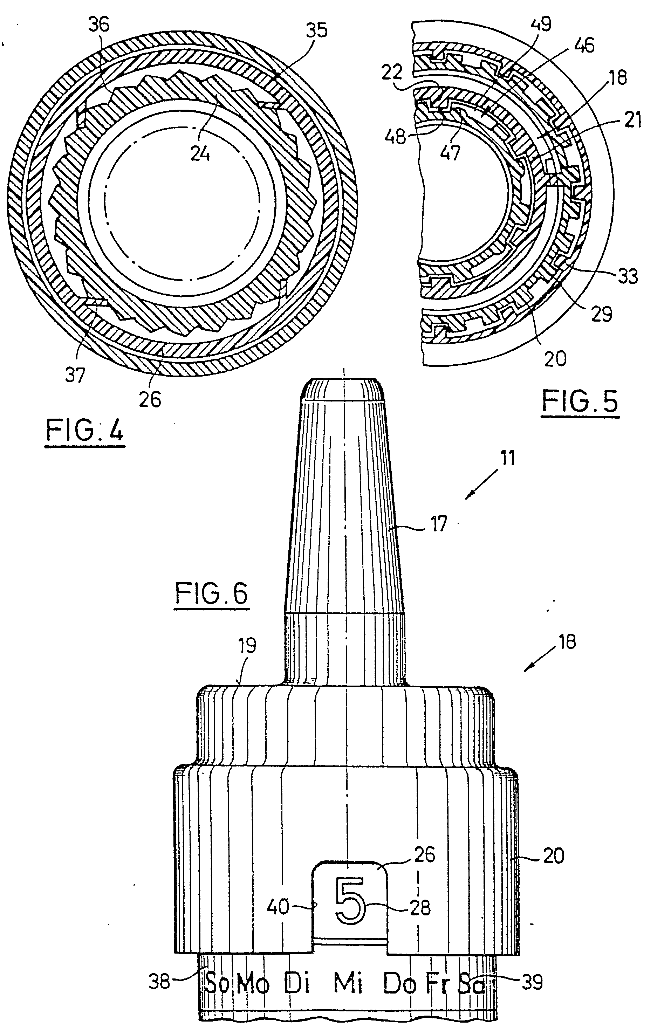

Der Basisteil besteht, wie alle übrigen beschriebenen Teile der Zerstäuberpumpe, aus Kunststoffspritzguß und hat eine dreifach abgestufte, sich nach oben verjüngende Hülsenform. Im mittleren Bereich befindet sich das Befestigungs-Innengewinde 25 zum Aufschrauben auf den Behälter 12. Die Außenseite der mittleren Stufe des als Deckelteil ausgebildeten Basisteiles 24 ist von einem Zählring 26 umgeben, der an dem Basisteil 24 durch Einschnappen eines Ringvorsprunges 27 in eine Ringnut axial festgelegt, jedoch drehbar ist. Der Zählring 26 trägt an seiner Außenseite, in einem unteren, etwas nach außen vorspringenden Bereich seines Mantels Schriftzeichen einer Zählbeschriftung 28, beispielsweise eine fortlaufende Numerierung, die der Zahl der Betätigungshübe entspricht, die die Zerstäuberpumpe ausführen soll (Fig. 6). Im oberen Teil hat der Zählring 26 an der Außenseite im wesentlichen axial verlaufende Rippen 29, die zu einem Fortschaltwerk 30 gehören und um den gesamten Umfang herum angeordnet sind. Die Rippen 29 haben an ihrer freien Oberseite abgeschrägte Schaltflächen 31 (Fig. 2), denen in entsprechender Weise abgeschrägte Schaltflächen 32 an inneren Rippen 33 im Betätigungsdrücker 18 zugeordnet sind. Aus Fig. 2 ist zu erkennen, daß die Zahl der Rippen 29 doppelt so groß ist, wie die der Rippen 33.

Ebenfalls zum Fortschaltwerk 30 gehört ein Gesperre 35 (Fig. 4). Es besteht aus einem Raststern 36 in Form einer äußeren Verzahnung des Basisteils 24, mit dem vier federnde Kunststofflaschen 37 des Zählringes 26 zusammenwirken. Die Kunststofflaschen 37 arbeiten mit dem Raststern 36 nach Art von Schaltklinken zusammen. Durch die Form der Verzahnung, die relativ flach geneigte Zahnflanken mit einem unsymmetrischen Einschnitt am Zahngrund aufweist, ist sichergestellt, daß die schräg angeordneten Kunststofflaschen 37 durch ihre Eigenfederung den Zählring 26 selbsttätig in die in Fig. 4 dargestellte Position drehen, und zwar auch aus einer gegenüber der dargestellten Lage um einige Grad verdrehten Stellung. Der Zählring 26 kann sich im Uhrzeigersinn drehen, wird jedoch entgegen dem Uhrzeigersinn an einer Drehung durch die Kunststofflaschen 37 gehindert.

Auf dem Basisteil 24 ist ferner ein Zusatzring 38 drehbar gelagert, der von Hand axial nach oben verschiebbar ist und dann durch eine entsprechende Verzahnung in den Zählring 26 eingekuppelt wird, so daß er sich zusammen mit dem Zählring dreht. Auch dieser Zusatzring 38 kann eine Beschriftung 39 tragen, z.B. in Form der Wochentage (Fig. 6). Das jeweils gültige Schriftzeichen auf dem Zählring 26 kann durch eine beliebige Kennzeichnung an dem Betätigungsdrücker 18 bezeichnet sein, vorzugsweise jedoch durch einen fensterartigen Ausschnitt bzw. ein Fenster 40 im Mantel 20.

Der Betätigungsdrücker 18 ist gegenüber dem Zählring 26 gegen Abziehen nach oben durch eine federnde Schnapp-Rastnase 41 gesichert.

Der Zählring 26 hat als Anschlag 42 an seiner oberen Innenseite einen nach innen ragenden Vorsprung, der in der Umfangsbahn eines entsprechenden Vorsprunges bzw. Gegenanschlages 43 des Betätigungsdrückers 18 liegt. Die Vorsprünge sind in Axialrichtung so zueinander bemessen, daß sie, wenn sie nicht zusammenarbeiten, die Axialbewegung des Betätigungsdrückers 18 gegenüber dem Zählring 26 bzw. dem Basisteil 24 nicht behindern, jedoch in der in Fig. 3 dargestellten Lage, in der ein als Anschlagfläche 44 vorgesehener Absatz des Anschlages 42 mit dem Gegenanschlag 43 zusammenwirkt, die Axialbewegung verhindern. Ein über die Anschlagfläche 44 nach oben reichender Drehsperr-Anschlag 45 des Anschlages 42 verhindert eine weitere Drehung des Zählringes über die in Fig. 3 gezeigte Sperrstellung hinaus. Die Anschläge 42, 43 bilden also eine Hubsperre für den Betätigungshub der Zerstäuberpumpe.

Aus Fig. 5 ist zusätzlich noch zu erkennen, daß eine Transportsicherung 46 vorgesehen ist, die bei einer gegenüber der in Fig. 5 dargestellten Betätigungsstellung vorgenommenen Verdrehung des Betätigungsdrückers 18 gegenüber dem Basisteil 24 im Uhrzeigersinne wirksam wird und eine Betätigung verhindert. Dabei verschieben sich die Zähne der Verzahnung 22 am Betätigungsdrücker 18 unter Überwindung eines buckelartigen Vorsprunges 47 in eine Stellung, in der sie nicht mehr in der ihnen entsprechenden Nut 48 laufen, sondern von einer Sperrfläche 49 an einer Axialbewegung gehindert werden. Die buckelartigen Vorsprünge sichern die entsprechende Transport- und Betätigungsstellung gegen versehentliche Verdrehung.

Die Funktion der Ausführungsform nach den Fig. 1 bis 6 ist wie folgt: Die Dosiereinrichtung 11 ist normalerweise fest auf dem Behälter 12 montiert, der mit der entsprechenden Menge eines auszugebenden Stoffes gefüllt ist. Es kann sich dabei um Flüssigkeit handeln, jedoch auch um andere fließfähige Stoffe bis zur Brei- oder Cremeform, die dann nicht zerstäubt, sondern lediglich an einer Auslaßöffnung ausgegeben werden. Es wird dafür gesorgt, daß die Pumpenkammer bereits gefüllt ist, so daß schon beim ersten Betätigungshub eine Ausgabe erfolgt. Es wäre jedoch auch möglich, bewußt einige anfängliche Ansaug-Hübe vorzusehen, bevor die eigentliche Zählung beginnt.

Zum Transport ist die Zerstäuberpumpe dadurch gesichert, daß gegenüber der in Fig. 5 gezeigten Stellung der Betätigungsdrücker 18 etwas im Uhrzeigersinne verdreht ist, so daß seine Verzahnung 22 oder seine Rippen über der Sperrfläche 49 stehen. Zur Inbetriebnahme wird der Betätigungsdrücker 18 entgegen dem Uhrzeigersinn verdreht, und er steht dann in der Stellung, die aus Fig. 5 ersichtlich ist. Wenn der Behälter beispielsweise mit einem Pharmazeutikum gefüllt ist, das in die Nasenöffnung zu applizieren ist, so wird der Schaft 17 in eine Nasenhöhle eingeführt und die Pumpe durch Druck auf die Schulter 19 betätigt. Die Verzahnung 22 läuft dabei in den Nuten 48 und behindert die Axialbewegung während des Betätigungshubes nicht, sichert den Betätigungsdrücker 18 jedoch gegen Verdrehung. Gleichzeitig kamen jedoch die Schrägflächen 31, 32 der Rippen 29, 33 miteinander in Eingriff und transportierten den Zählring 26 in Fig. 2 nach links, und zwar um eine Strecke, die etwas geringer ist, als der Abstand zwischen den Rippen 29. Dabei werden auch die Kunststofflaschen 37 jeweils über die Scheitel des an sie angrenzenden Zahns des Raststerns 36 transportiert. Beim Rückwärtshub nach erfolgter Zerstäubung des auszugebenden Stoffes durch die Zerstäuberdüse 16 kommen die beiden Rippen 29, 33 wieder außer Eingriff, und die Kunststofflaschen 37 laufen auf den schrägen Zahnflanken abwärts und drehen den Raststern 36 weiter in eine Lage entsprechend Fig. 4. In dieser Lage stehen dann die nächstfolgende Zahl der Zählbeschriftung 28 und die nächstfolgende Beschriftung 39 auf dem Zusatzring 38 im Bereich des Fensters 40.

Der Zusatzring 38 war gegenüber der in Fig. 1 gezeigten Stellung aufwärts verschoben worden, und zwar in eine Stellung, in der die Beschriftungen 28, 39 zueinander passen (beispielsweise Datum und Wochentag).

Dieser Zyklus wird so lange wiederholt, bis die Anschläge 42 und Gegenanschläge 43 sich in der Lage nach Fig. 3 befinden. Dies erfolgt nach einer Zahl von Betätigungshüben, die dem Benutzungszyklus entspricht. Bei einer dreiwöchigen Benutzungsdauer würde beispielsweise nach dem 21. Hub diese Stellung erreicht sein und die Pumpe wäre gegen weitere Benutzung gesperrt. Da bei der gezeigten Ausführungsform keine Handhabe gezeigt ist, um die Pumpe wieder zurückzustellen, wäre damit die Benutzungsdauer beendet. Eine versehentliche Weiterdrehung wird durch den Drehsperr-Anschlag 45 verhindert. Bei einem Pharmazeutikum, dessen Verwendung nach einer bestimmten Zeit wiederholt werden sollte, könnte jedoch dieser Anschlag 45 fehlen und stattdessen eine kräftige Rast vorgesehen sein, die durch manuelle Verdrehung des mit einer Handhabe versehenen Zählringes 26 überfahren werden könnte, so daß dann der Benutzungszyklus erneut beginnen könnte.

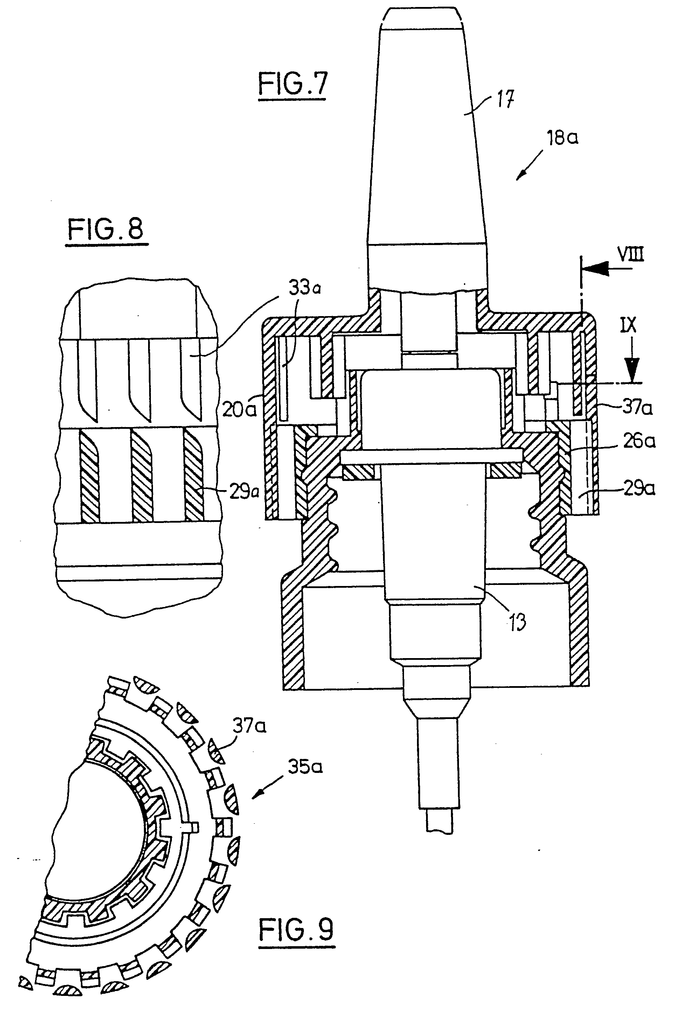

In den Fig. 7 bis 9 ist eine andere Ausführungsform gezeigt. In diesen Figuren tragen gleiche Teile gleiche Bezugszeichen, die bei ähnlichen Teilen durch den Index «a» ergänzt sind. Wegen ihrer Beschreibung im einzelnen wird zur Vermeidung von Wiederholungen auf das Vorstehende Bezug genommen.

Bei dieser Ausführung sind der Mantel 20a des Betätigungsdrückers 18a und der Zählring 26a axial kürzer ausgeführt. Die Rippen 29a am Zählring 26a sind in radialer Richtung stärker ausgeführt und arbeiten außer mit den als einzelne freistehende axial verlaufende Stege ausgebildeten Weiterschalterippen 33 an ihrem Außenumfang mit Rastfedern 37a zusammen, die als blattfederartige Streifen geformt und durch axial verlaufende Einschnitte im Mantel 20a gebildet sind. Die Zahl dieser als Kunststoff-Federlaschen ausgebildeten Rastfedern 37a entspricht der Zahl der Rippen 29a, so daß sie dadurch ein Gesperre 35a bilden.

Diese Ausführung ist besonders einfach. In der dargestellten Ausführungsform ist jedoch keine Sicherung gegen Rückdrehung vorhanden. Diese könnte jedoch durch eine unsymmetrische Gestaltung der Rastfedern 37a erfolgen. Wie das Gesperre 35 bei der Ausführungsform nach Fig. 1 bis 6 übernimmt auch das Gesperre 35a die Aufgabe der Weiterschaltung bis in eine definierte Drehstellung bei jedem Hub. Zum Erreichen einer guten Federwirkung befindet sich innerhalb der Rastfedern 37a ein Einstich, der sie von den Rippen 33a trennt. Diese Ausführungsform hat bei im übrigen gleicher Gestaltung keinen Zusatzring.

**Patentansprüche**

1. Betätigbare Dosiereinrichtung mit einem Betätigungsdrücker (18, 18a) zur Ausgabe einer Mengeneinheit eines fließfähigen Stoffes bei jedem Betätigungshub aus einem Behälter (12) und mit einer, insbesondere einen Zählring (26, 26a) aufweisenden, Zähleinrichtung für die Betätigungshübe, gekennzeichnet durch eine in Abhängigkeit von der Zähleinrichtung betätigbare Hubsperre (42, 43) für den Betätigungshub.

2. Dosiereinrichtung nach Anspruch 1, dadurch gekennzeichnet, daß die Hubsperre einen über ein Fortschaltwerk (30) der Zähleinrichtung von mindestens einer Ausgangsstellung über Freigabestellungen in eine dem Ende eines mehrhubigen Benutzungszyklus' zugehörige Sperrlage bewegten, insbesondere an einem Innenumfang liegenden, Anschlag (42) aufweist, dem ein Gegenanschlag (43) des Betätigungsdrückers (18) zugeordnet ist, welcher vorzugsweise in einer Sperr-Drehlage (Fig. 3) von dem Anschlag (42) gegen Axialbewegungen gesperrt ist.

3. Dosiereinrichtung nach Anspruch 2, dadurch gekennzeichnet, daß der Anschlag (42) am Zählring (26), insbesondere in Form eines Vorsprunges vorgesehen ist und vorzugsweise am äußeren Ende des Zählringes (26) liegt.

4. Dosiereinrichtung nach Anspruch 2 oder 3, dadurch gekennzeichnet, daß der Anschlag (42) in der Sperr-Drehlage (Fig. 3) gegen Weiterdrehung durch eine Drehsperre gesperrt ist, wobei vorzugsweise eine Anschlagfläche (44), insbesondere die Anschlagfläche (44) des Anschlages (42), nach Art einer abgesetzten Schulter in einen über sie vorstehenden Drehsperr-Anschlag (45) bzw. eine Drehsperr-Rast übergeht.

5. Dosiereinrichtung nach einem der vorhergehenden Ansprüche, dadurch gekennzeichnet, daß die Hubsperre lösbar ausgebildet ist und insbesondere nach einer Skalierung (28, 39) in unterschiedliche Dosierungen überführbar ist.

6. Dosiereinrichtung nach einem der Ansprüche 2 bis 5, dadurch gekennzeichnet, daß der Anschlag (42) gegenüber dem Gegenanschlag (43), insbesondere mit einer Handhabe, aus der Sperrlage in eine Ausgangslage für einen weiteren Benutzungszyklus überführbar ist, wobei vorzugsweise die Drehsperr-Rast manuell überfahrbar ausgebildet ist.

7. Dosiereinrichtung nach einem der vorhergehenden Ansprüche, dadurch gekennzeichnet, daß die Zähleinrichtung gegen Rückdrehung, insbesondere durch ein Freilauf-Gesperre (35), gesperrt ist, das vorzugsweise durch das Fortschaltwerk (30) gebildet ist.

8. Dosiereinrichtung nach einem der Ansprüche 2 bis 7, dadurch gekennzeichnet, daß das Fortschaltwerk (30) abgeschrägte Schaltflächen (31, 32) und vorzugsweise eine Einrasteinrichtung aufweist, die den Anschlag (42) bzw. den Zählring (26) jeweils nach Überschreiten einer vorgegebenen Drehstellung jeweils in eine bestimmte Lage weiterdreht, wobei insbesondere das Freilauf-Gesperre (35) aus einem Raststern (36) und federnden, angeformten Kunststofflaschen (37) besteht.

9. Dosiereinrichtung nach einem der vorhergehenden Ansprüche, dadurch gekennzeichnet, daß ein beispielsweise der Befestigung am Behälter (12) dienender Basisteil (24) vorgesehen ist, gegenüber welchem der Betätigungsdrücker (18) axial bewegbar und auf dem der ihn umgebende Zählring (26) drehbar gelagert ist, daß insbesondere das bei Betätigung schrittweise arbeitende Fortschaltwerk (30) zwischen dem Betätigungsdrücker (18) und dem Zählring (26) vorgesehen ist und daß vorzugsweise der Betätigungsdrücker (18) mit dem Basisteil (24) drehfest, jedoch axial beweglich verbunden ist.

10. Dosiereinrichtung nach einem der vorhergehenden Ansprüche, dadurch gekennzeichnet, daß ein Zusatzring (38) vorgesehen ist, der insbesondere durch Axialverschiebung in den Zählring (26) einkuppelbar ist, wobei insbesondere der Betätigungsdrücker (18) einen den Zählring (26) übergreifenden Mantel (20) mit einem einen Teil einer Zählbeschriftung (28) freigebenden Fenster (40) aufweist.

11. Dosiereinrichtung nach einem der vorhergehenden Ansprüche, gekennzeichnet durch eine durch Verdrehung des Betätigungsdrückers (18) gegenüber der übrigen Dosiereinrichtung betätigbare Transportsicherung, die die Durchführung eines Betätigungshubes verhindert.

12. Dosiereinrichtung nach einem der vorhergehenden Ansprüche, dadurch gekennzeichnet, daß sie eine, insbesondere als Zerstäuber ausgebildete, Austragpumpe (11) aufweist, die vorzugsweise mit einem mit dem Betätigungsdrücker (18) verbundenen Kolben versehen ist, der einer mit dem auszutragenden Stoff gefüllten Pumpenkammer zugeordnet ist.

**Claims**

1. Actuatable dosing mechanism with an actuating pusher (18, 18a) for dispensing a quantity unit of a flowable substance during each actuating stroke from a container (12) and with a counting device for the actuating strokes, in particular having a counting ring (26, 26a), characterized by a stroke stop (42, 43) for the actuating stroke operable as a function of the counting device.

2. Dosing mechanism according to claim 1, characterized in that the stroke stop has a stop member (42), particularly located on an internal circumference and moved by means of a stepping device (30) of the counting device from at least one starting position via release positions into a stopping position associated with the end of a multistroke use cycle and with which is associated a counter-stop member (43) of the actuating pusher (18), which is preferably locked in a locking rotation position (fig. 3) by stop member (42) against axial movements.

3. Dosing mechanism according to claim 2, characterized in that the stop member (42) is located on counting ring (26), particularly in the form of a projection and is preferably positioned at the outer end of counting ring (26).

4. Dosing mechanism according to claims 2 or 3, characterized in that the stop member (42) is locked in the locking rotation position (fig. 3) against further rotation by a rotation preventing means and preferably a stop face (44), particularly that of the stop member (42) passes in the manner of an offset shoulder into a rotation preventing stop member (45) or a rotation preventing detent projecting over the same.

5. Dosing mechanism according to one of the preceding claims, characterized in that the stroke stop is detachably constructed and can be transferred into different dosages by means of a scala (28, 39).

6. Dosing mechanism according to one of the claims 2 to 5, characterized in that the stop (42) with respect to the counter-stop (43), particularly by means of a handle, can be transferred from the locking position into a starting position for a further use cycle and preferably it is possible to manually overcome the rotation preventing detent.

7. Dosing mechanism according to one of the preceding claims, characterized in that the counting device is blocked against rotating back, particularly by a safety catch (35), which is preferably formed by the stepping mechanism (30).

8. Dosing mechanism according to one of the claims 2 to 7, characterized in that the stepping mechanism (30) has chamfered operating faces (31, 32) and preferably a locking device, which further rotates into a given position the stop member (42) or the counting ring (26) in each case after exceeding a given rotation position and in particular the safety catch (35) comprises a notched star (36) and resilient, plastic, shaped-on strips (37).

9. Dosing mechanism according to one of the preceding claims, characterized in that a base part (24) e.g. used for fixing to the container (12) is provided and with respect to which the actuating pusher (18) is axially movable and on which is rotatably mounted the counting ring (26) surrounding the same, that in particular the stepping mechanism (30) operating in stepwise manner on operation is provided between the operating pusher (18) and the counting ring (26) and that preferably the operating pusher (18) is connected in non-rotary, but axially movable manner to the base part (24).

10. Dosing mechanism according to one of the preceding claims, characterized in that an additional ring (38) is provided, which can in particular be coupled by axial displacement into the counting ring (26) and in particular the actuating pusher (18) has a jacket (20) engaging over the counting ring (26) with a window (40) freeing part of the counting inscriptions (28).

11. Dosing mechanism according to one of the preceding claims, characterized by a transport securing means operable by rotating the actuating pusher (18) with respect to the remaining dosing mechanism and which prevent the performance of an operating stroke.

12. Dosing mechanism according to one of the preceding claims, characterized in that it has a discharge pump (11), particularly constructed as an atomizer, which is preferably provided with a piston connected to the actuating pusher (18) and with which is associated a pump chamber filled with the substance to be discharged.

**Revendications**

1. Dispositif de dosage susceptible d'être commandé, avec un poussoir de commande (18, 18a) pour débiter une quantité unitaire d'une substance capable de s'écouler, lors d'une course de commande, à partir d'un récipient (12) et avec un dispositif de comptage des courses de commande, présentant en particulier un anneau de comptage (26, 26a), caractérisé par un arrêt de course (42, 43) actionnable en dépendance du dispositif de comptage, pour la course de commande.

2. Dispositif de dosage suivant la revendication 1, caractérisé en ce que l'arrêt de course comporte une butée (42) se trouvant en particulier sur un pourtour intérieur, déplacée par l'intermédiaire d'un mécanisme d'avancement (30) du dispositif de comptage, à partir d'au moins une position initiale en passant par des positions de libération, dans une position d'arrêt correspondant à la fin d'un cycle d'utilisation à courses multiples, butée à laquelle est associée une butée coopérante (43) du poussoir de commande (18), qui est bloquée à l'encontre de mouvements axiaux, de préférence dans une position d'arrêt de rotation (figure 3), par la butée (42).

3. Dispositif de dosage suivant la revendication 2, caractérisé en ce que la butée (42) est prévue sur l'anneau de comptage (26), en particulier en forme d'une saillie, et se trouve de préférence à l'extrémité extérieure de l'anneau de comptage (26).

4. Dispositif de dosage suivant la revendication 2 ou 3, caractérisé en ce que la butée (42) est bloquée dans la position d'arrêt de rotation (figure 3), en empêchant la poursuite de la rotation, par un arrêt de rotation, où, de préférence, une surface de butée (44), en particulier la surface d'application (44) de la butée (42), à la manière d'un épaulement décalé, passe à une butée (45) d'arrêt de rotation, saillante au-dessus d'elle, respectivement à un cran de blocage de rotation.

5. Dispositif de dosage suivant l'une des revendications précédentes, caractérisé en ce que l'arrêt de course est réalisé libérable et peut être transféré à des dosages différents, en particulier d'aprs une graduation (28, 39).

6. Dispositif de dosage suivant l'une des revendications 2 à 5, caractérisé en ce que la butée (42) peut être transférée par rapport à la butée coopérante (43), en particulier à l'aide d'une poignée, de la position de blocage à une position de départ pour un nouveau cycle d'utilisation, le cran d'arrêt de rotation étant transférable de préférence à la main.

7. Dispositif de dosage suivant l'une des revendications précédentes, caractérisé en ce que le dispositif de comptage est bloqué en empêchant une rotation en arrière, en particulier par un dispositif d'arrêt libre (35) qui est formé de préférence par le mécanisme d'avancement (30).

8. Dispositif de dosage suivant l'une des revendications 2 à 7, caractérisé en ce que le mécanisme d'avancement (30) comporte des surfaces de positionnement biseautées (31, 32) et en particulier un dispositif d'encliquetage qui continue à faire tourner la butée (42), respectivement l'anneau de comptage (26), chaque fois après dépassement d'une position de rotation prédéterminée, à une position déterminée, le dispositif d'arrêt libre (35) consistant en particulier en une étoile à crans (36) et en des brides de matière synthétique élastiques (37) formées sur l'anneau de comptage.

9. Dispositif de dosage suivant l'une des revendications précédentes, caractérisé en ce qu'est prévue une pièce de base (24) servant par exemple à la fixation sur un récipient (12), pièce par rapport à laquelle le poussoir de commande (18) est déplaçable axialement, et sur laquelle est monté, de façon à pouvoir tourner, l'anneau de comptage (26) qui l'entoure; en ce que, en particulier, le mécanisme d'avancement (30) fonctionnant pas à pas lorsqu'il est commandé, est prévu entre le poussoir de commande (18) et l'anneau de comptage (26); et en ce que, de préférence, le poussoir de commande (18) est relié solidairement en rotation à la pièce de base (24) mais en pouvant se déplacer axialement.

10. Dispositif de dosage suivant l'une des revendications précédentes, caractérisé en ce qu'est prévu un anneau supplémentaire (38) qui peut être accouplé, en particulier par déplacement axial, à l'anneau de comptage (26), où le poussoir de commande (18), en particulier, présente une enveloppe (20) surplombant l'anneau de comptage (26), avec une fenêtre (40) dégageant une partie d'une inscription de comptage (28).

11. Dispositif de dosage suivant l'une des revendications précédentes, caractérisé par un dispositif de sûreté pour le transport, actionnable par une rotation du poussoir de commande (18) par rapport au reste du dispositif de dosage, cette sûreté empêchant l'exécution d'une course de commande.

12. Dispositif de dosage suivant l'une des revendications précédentes, caractérisé en ce qu'il comporte une pompe de débit (11), en particulier conformée comme pulvérisateur, qui est de préférence pourvue d'un piston relié au poussoir de commande (18), ce piston étant associé à une chambre de pompe remplie de la substance à débiter.

FIG.1

FIG.2

FIG.3

FIG.4

FIG.5

FIG.6

So Mo Di Mi Do Fr Sa

FIG.7

FIG.8

FIG.9